# EUROPEAN PATENT SPECIFICATION

(11) **EP 3 801 215 B1**
(45) Date of publication and mention of the grant of the patent: **14.10.2026**
(21) Application number: 19810364.0
(22) Date of filing: 24.05.2019
(51) Int. Cl.: A61B 5/00, A61B 5/01, A61B 8/12, G01N 23/04, G05B 23/02, G06T 7/00, G06T 11/00, G16H 30/00, G16H 10/60, G16H 30/20, G16H 50/20

(54) **INFRARED THERMOGRAPHY PLATFORM FOR DETERMINING VASCULAR HEALTH OF INDIVIDUALS**
INFRAROT-THERMOGRAFIEPLATTFORM ZUR BESTIMMUNG DER GEFÄSSGESUNDHEIT VON PERSONEN
PLATEFORME DE THERMOGRAPHIE INFRAROUGE POUR DÉTERMINER LA SANTÉ VASCULAIRE D'INDIVIDUS

(30) Priority: 30.05.2018 US 201862677887 P
(43) Date of publication of application: 14.04.2021
(73) Proprietor: USA Therm, Inc., Hollywood. FL 33020 (US)
(72) Inventor: SOFFER, Ariel, Aventura, Florida 33180 (US); WRIGHT, David, Oxfordshire RG9 1LP (GB)
(74) Representative: AWA Sweden AB
(86) International application number: PCT/US2019/033911
(87) International publication number: WO 2019/231842

(56) References cited:
- WO-A1-2018/002863
- WO-A1-2018/002863
- WO-A1-2018/002925
- WO-A1-2018/002925
- US-A1- 2004 073 132
- US-A1- 2010 172 567
- US-A1- 2010 191 541
- US-A1- 2013 116 573
- US-A1- 2014 164 968
- US-A1- 2017 277 831
- US-A1- 2018 082 016
- US-A1- 2018 098 727
- KIRIMTAT AYCA ET AL: "FLIR vs SEEK in Biomedical Applications of Infrared Thermography", 28 March 2018, ADVANCES IN DATABASES AND INFORMATION SYSTEMS; [LECTURE NOTES IN COMPUTER SCIENCE; LECT.NOTES COMPUTER], SPRINGER INTERNATIONAL PUBLISHING, CHAM, PAGE(S) 221 - 230, ISBN: 978-3-319-10403-4, XP047502048
- CHOLEWKA ARMAND ET AL: "How to use thermal imaging in venous insufficiency?", JOURNAL OF THERMAL ANALYSIS AND CALORIMETRY, KLUWER, DORDRECHT, NL, vol. 130, no. 3, 21 February 2017 (2017-02-21), pages 1317 - 1326, XP036364956, ISSN: 1388-6150, [retrieved on 20170221], DOI: 10.1007/S10973-017-6141-7

## Description

### Technical Field

The present disclosure relates to vascular medicine,
and more particularly, to the use of infrared thermography for determining the vascular health of individuals.

### Background

Venous insufficiency is a common condition that manifests itself in a very broad range from invisible vein incompetence to massive varicose veins and ultimately ulcers. In excess of 40% of the adult population are affected to some extent.

Improper functioning of the vein valves in the leg, for example, can cause swelling and skin changes. Normally, the vein valves keep blood moving back toward the heart. Venous valvular failure leads to a reverse blood flow in the veins. In addition to swelling or skin color changes, varicose veins may form. If the condition progresses, leg ulcers may form. Treatment includes compression stockings, surgery, foam sclerotherapy, heat ablation and glue closure, for example.

If not treated, the pressure and swelling may increase until the tiniest blood vessels in the legs (capillaries) burst. When this happens, the overlying skin takes on a reddish-brown color and is very sensitive to being broken if bumped or scratched.

Tests called a venogram or a duplex ultrasound may be used to examine the blood circulation in a patient. During a venogram, an intravenous (IV) x-ray contrast solution is placed into the veins. The contrast solution causes the blood vessels to appear white on the X-ray image, which helps the doctor see them on the image. The contrast will provide a clear X-ray picture of the blood vessels which would otherwise be invisible.

The duplex ultrasound may be used to identify the veins and arteries and test the speed and direction of blood flow in the veins. A technician will place gel on the skin of the patient and then press a small hand-held device (transducer) against the skin. The transducer sends sound waves that bounce back off tissues and vessels to the transducer. The sound waves are analyzed, and an image of the vessel is created and displayed on a monitor.

Even in view of the current tests used to examine blood flow circulation, there is still a need for a non-invasive and straightforward screening methodology to assess the vascular health of individuals.

The paper "How to use thermal imaging in venous insufficiency" (Armand Cholweka, JOURNAL OF THERMAL ANALYSIS AND CALORIMETRY, KLUWER, DORDRECHT, NL, vol. 130, no. 3, 21 February 2017 (2017-02-21), pages 1317-1326) describes studies on the usefulness of thermal imaging for the insufficiency of superficial veins in the lower limbs. It generates thermograms after subtraction of areas characterised with lower temperature than isotherm.

### Summary

The object of the present invention is achieved with a system according to claim 1. Further preferred embodiments are described in the dependent claims.

A vascular thermography system includes at least one portable electronic device and a thermal image analyzer in communications therewith. The portable electronic device includes a thermal imager configured to generate a thermal image of an anatomical area of a patient, a display, a processor configured to display the thermal image on the display, and a transceiver coupled to the processor and configured to transmit the thermal image. The thermal image analyzer is configured to receive the thermal image from the portable electronic device, determine an assessment on vascular health of the patient based on comparing the thermal image to a database of thermal images, and transmit the assessment to the portable electronic device.

The vascular thermography system advantageously allows for real-time clinical assessments on the vascular health of patients. By using thermography and an intelligent database this allows for real-time clinical assessments to be performed non-invasively and without requiring expensive ultrasound equipment.

The vascular thermography system may further comprising a remote storage device associated with the thermal image analyzer. The remote storage device may be configured to store a patient list and cataloged thermal images of patients on the patient list. The processor may be further configured to execute a clinical thermography application to display a login screen, and display vein scan settings based on a user of the portable electronic device logging in. The vein scan settings include at least one of a live view setting allowing the user to immediately examine a patient, a new patient setting for the user to add a new patient to the patient list, and an existing patient setting for the user to retrieve an existing patient from the patient list.

The processor may be further configured to display a drop down list so the user can label the anatomical area being displayed in the thermal image, and store the labeled thermal image in the cataloged thermal images at the remote storage device, based on execution of the clinical thermography application.

The thermal image analyzer and the remote storage device may be cloud-based. Communications between the portable electronic device and both the thermal image analyzer and the remote storage device may be encrypted so as to be HIPPA (Health Insurance Portability and Accountability) compliant.

The database of thermal images may comprise a plurality of thermal patterns corresponding to a range of vascular health conditions, and wherein the thermal image analyzer may be further configured to compare a thermal pattern within the thermal image to the plurality of thermal patterns in the database of thermal images. The assessment on vascular health of the patient may include statistical probabilities of certain disease states based on the thermal pattern within the thermal image.

The thermal image analyzer may be further configured to use machine learning to determine the assessment on vascular health of the patient.

The portable electronic device may further comprise an external interface coupled to the processor, and a housing. The housing is configured to carry the display, the processor, the transceiver, and the external interface. The thermal imager may be external the housing and removeably coupled to the external interface.

The portable electronic device may further comprises a camera configured to take a photograph of the same anatomical area as being displayed in the thermal image. The processor may be further configured to display in a side-by-side comparison the thermal image and the photograph of the same anatomical area.

Another aspect is directed to a vascular thermography system that includes at least one portable electronic device comprising a thermal imager configured to generate a thermal image of an anatomical area of a patient, a display, a transceiver, and a processor coupled to the thermal imager, the display, and the transceiver. The processor may be configured to execute a clinical thermography application to display the thermal image, display a drop down list so the user can label the anatomical area being displayed in the thermal image, and transmit the labeled thermal image. The vascular thermography system may further include a remote storage device configured to store the labeled thermal image as a cataloged thermal image of the patient, and a thermal image analyzer associated with the remote storage device. The thermal image analyzer may be configured to receive the cataloged thermal image from the remote storage device, determine an assessment on vascular health of the patient based on comparing the cataloged thermal image to a database of thermal images, and transmit the assessment to the at least one portable electronic device.

Yet another aspect is directed to a method for operating a vascular thermography system comprising at least one portable electronic device and a thermal image analyzer. The portable electronic device includes a thermal imager, a display, a transceiver, and a processor coupled to the thermal imager, the display, and the transceiver. The method comprises operating the at least one portable electronic device to generate a thermal image of an anatomical area of a patient for display, and transmit the thermal image to the thermal image analyzer. The method further includes operating the thermal image analyzer may be operated to receive the thermal image from the at least one portable electronic device, determine an assessment on vascular health of the patient based on comparing the thermal image to a database of thermal images, and transmit the assessment to the at least one portable electronic device.

### Brief Description of the Drawings

FIG. 1 is a block diagram of a clinical based infrared thermography system in accordance with the disclosure.
FIG. 2 is a flow diagram for operating the clinical based thermography application illustrated in FIG. 1.
FIG. 3-9 are various display screens corresponding to the flow diagram illustrated in FIG. 2.
FIG. 10 is a flowchart illustrating a method for operating the infrared thermography system as illustrated in FIG. 1.
FIG. 11 is a block diagram of a retail based infrared thermography system in accordance with the disclosure.

### Detailed Description

The present description is made with reference to the accompanying drawings, in which exemplary embodiments are shown. However, many different embodiments may be used, and thus the description should not be construed as limited to the particular embodiments set forth herein. Rather, these embodiments are provided so that this disclosure will be thorough and complete. Like numbers refer to like elements throughout.

Infrared thermography platforms as described below for determining vascular health of individuals may be configured for clinical use or for retail use. Clinical use of an infrared thermography platform involves a doctor operating a portable vascular thermography device that is in communications with a cloud based thermal image analyzer to assess the vascular health of a patient. Retail use of an infrared thermography platform involves an individual interacting with a kiosk to assess the vascular health of a customer so as to recommend appropriately sized and strength of compression socks as necessary.

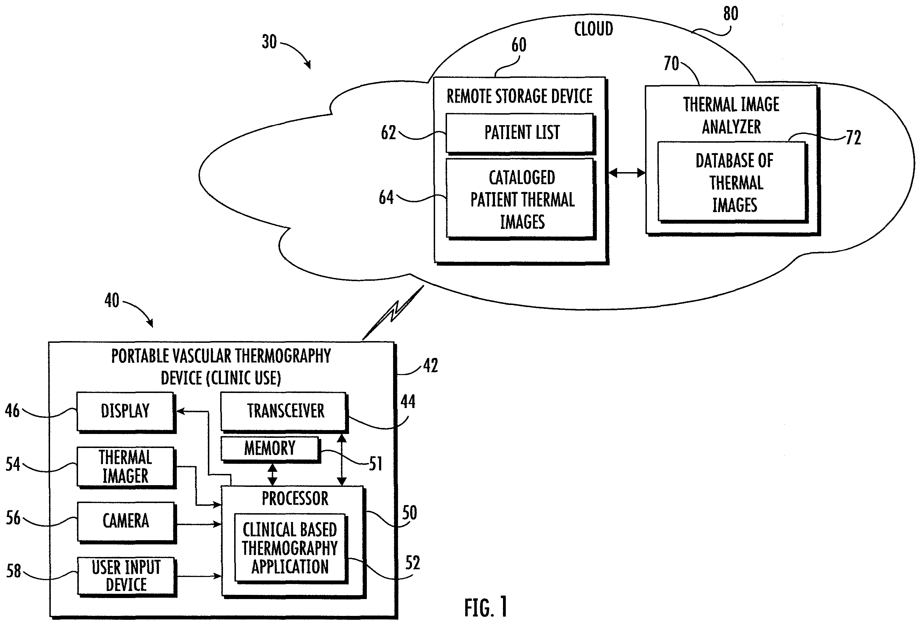

Referring initially to FIG. 1, a clinical based infrared thermography system **30** includes at least one portable vascular thermography device **40** operating a clinical based thermography application **50** in communications with a remote storage device **60** and a thermal image analyzer 70. The portable vascular thermography device **40** may also be referred to as a portable electronic device, and the clinical based infrared thermography system 30 may also be referred to as a vascular thermography system 30. The vascular thermography system **30** advantageously allows for real-time clinical assessments on the vascular health of individuals. By using thermography and an intelligent database this allows for real-time clinical assessments to be performed non-invasively and without requiring expensive ultrasound equipment.

As readily appreciated by those skilled in the art, infrared clinical thermography is a passive non-contact, and non-invasive method for mapping surface temperatures of the body. The differential of relative skin temperatures can be utilized to map and thus detect many abnormalities not seen by other diagnostic tools nor the human eye. The portable vascular thermography device **40** coupled with a validated database of thermal images **72** provides diagnostic immediacy that will enable physicians, nurses, and even consumers to quickly know more about their vascular health.

The remote storage device **60** and the thermal image analyzer **70** are cloud based 80. In other embodiments, the remote storage device **60** and the thermal image analyzer **70** may be co-located with the portable vascular thermography device **40.** Even though the remote storage device **60** and the thermal image analyzer **70** are shown as separate components, they may be combined into a single component.

Communications between the portable vascular thermography device **40** and the remote storage device **60** and the thermal image analyzer **70** are encrypted to be HIPPA (Health Insurance Portability and Accountability Act) complainant. To be HIPPA compliant, the clinical based infrared thermography system **30** includes encryption, an encrypted firewall, and role-based security to give users different levels of access based on their roles.

The remote storage device **60** is configured to store a patient list **62** and cataloged patient thermal images **64** as provided by the portable vascular thermography device 40. The thermal images are cataloged into corresponding labeled anatomical areas, such as left thigh, right calf, etc. The cataloging may be performed by the clinical based thermography application **50** or by the thermal image analyzer **70.**

The thermal image analyzer **70** is coupled to the remote storage device **60** and is configured to provide diagnosis or an assessment of the cataloged patient thermal images **64** by comparing these images to the database of thermal images **72.** The thermal image analyzer **70** may be configured to use machine learning to provide statistical probabilities of certain disease states based on the thermal patterns of the cataloged patient thermal images **64.**

The portable vascular thermography device **40** includes a housing **42,** a transceiver **44** carried by the housing **42** to communicate with the remote storage device **60** and the thermal image analyzer **70,** and a display **46** carried by the housing **42.**

The display **46** and the transceiver **44** are coupled to a processor **50** also carried by the housing **42.** The processor **50** is configured to execute a clinical based thermography application **52.**

A thermal imager **54** is carried by the housing **42** and is coupled to the processor 60. Optionally, the thermal imager **54** may be a separate component that is externally coupled to the portable vascular thermography device **40.** A memory **51** is coupled to the processor **50** for storing thermal images prior to the thermal images being stored in the remote storage device **60.**

In addition, a camera **56** may be carried by the housing **42** and coupled to the processor 60. The camera **56** allows for a comparison between a patient's thermal image and a standard photograph of the anatomical area being examined. A user input device **58** also carried by the housing **42** is coupled to the processor **50.**

The portable vascular thermography device **40** may be configured as an iPhone, iPod, iPad or Android device, for example. If the portable vascular thermography device **40** is configured as an iPhone, for example, then the thermal imager **54** may be separate from the iPhone and coupled thereto via the iPhone's USB interface. An example external plug-in thermal imager **54** is FLIR ONE Gen 3 as provided by FLIR Systems, Inc.

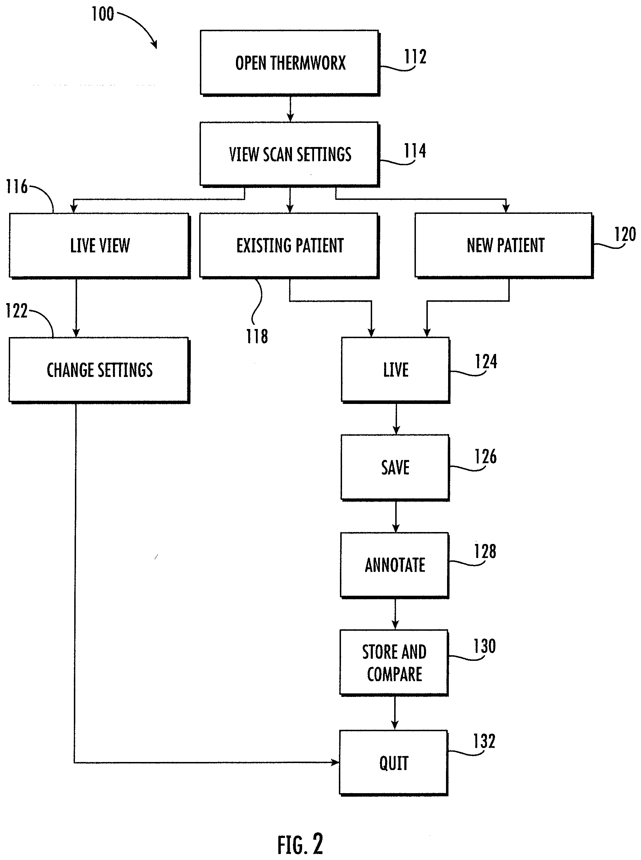

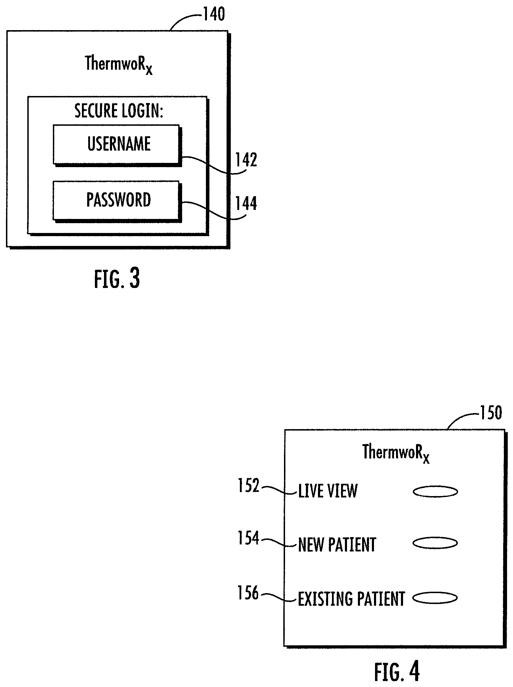

Referring now to the flow diagram **100** in FIG. 2 and to the display screens in FIGS. 3-9, operation of the infrared thermography application **50** by a doctor will be discussed. The clinical based thermography application **50** may also be referred to as Thermworx.

From the start, the clinical based infrared thermography application **50** is opened at Block **112** via a login screen **140** as shown in FIG. 3. The login screen **140** includes a username field **142** and a password field **144.** At Block **114** the available vein scan settings are provided via display screen **150** as shown in FIG. 4. The vein scan settings include a live view setting **152,** a new patient setting **154** and an existing patient setting **156.**

The live view setting **152** allows the doctor to immediately examine a patient at Block **116** regardless of whether the patient is an existing patient or a new patient. As the setting names imply, the existing patient setting **156** is for selecting an existing patient at Block **118** from the remote storage device **60,** and the new patient setting **154** is for entering a new patient at Block **120** into the remote storage device **60.**

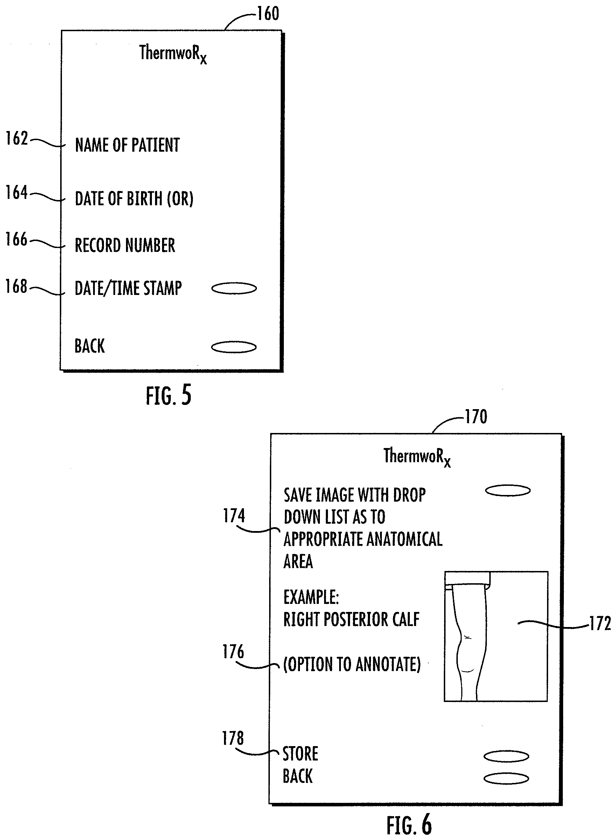

If the existing patient setting **156** is selected by the doctor, then display screen **160** appears as shown in FIG. 5. The doctor is to enter information into a name of patient field **162,** a date of birth field **164** or a record number field **166,** and a data/time stamp field **168.**

With the existing patient's information having been retrieved, the doctor may then use the thermal imager **54** to provide a thermal image **172** of the existing patient's right leg, for example, as shown in FIG. 6. The doctor has access to a save image field **174** for saving the thermal image **172.** The thermal image **172** is saved within the memory **51** prior to being stored in the remote storage device **60.**

Prior to the thermal image **172** being saved, the doctor selects from a drop down list to designate or label an appropriate anatomical area to the thermal image **172.** In this case, the appropriate anatomical area is right posterior calf.

The doctor has an option to annotate the thermal image via the option to annotate field **176.** If the doctor wants to store the annotated thermal image **172** within the remote storage device **60,** then the store field **178** is selected. The annotated thermal image **172** is stored within the cataloged patient thermal images **64.**

Referring back to the flow diagram **100,** after an existing patient **118** or a new patient **120** has been selected, the following steps are the same. These steps include providing the live thermal image **172** of the area being examined at Block **124,** saving the thermal image **172** corresponding to an appropriate anatomical area at Block **126,** an option to annotate the thermal image **172** at Block **128,** and storing the thermal image **172** within the remote storage device **60** at Block **130.**

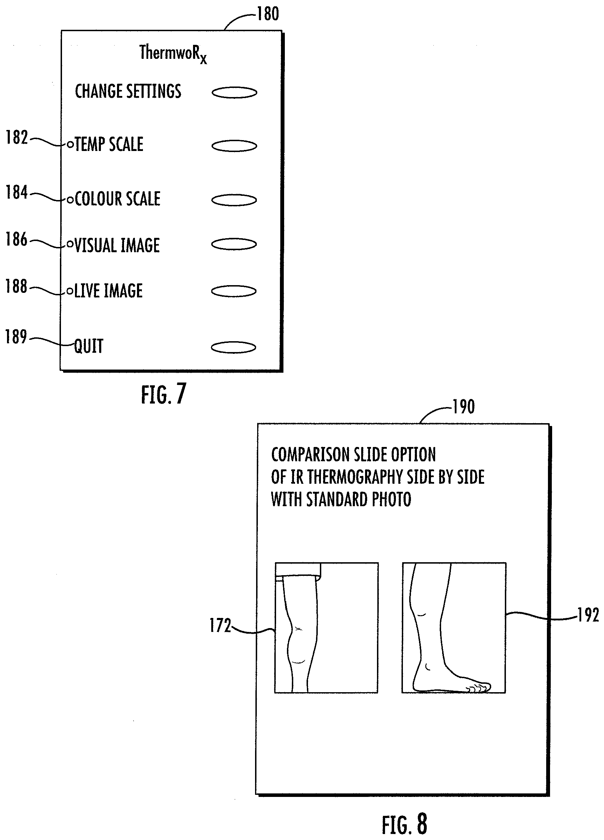

At Block **122** following the live view Block **116,** the settings may be changed as provided in display screen **180** shown in FIG. 7. The setting changes include a temperature scale field **182,** a color scale field **184,** a visual image field **186,** and a live image field **189.** The doctor can exit the display screen **180** by selecting the quit field **189.**

Still referring to the flow diagram **100,** when the thermal image **172** is stored within the remote storage device **60** at Block **130,** the thermal analyzer **70** may be instructed to compare the thermal image **172** to the database of thermal images **72.**

The thermal analyzer **70** may be configured to use machine learning to give the doctor statistical probabilities of certain disease states based on a thermal pattern within the thermal image **172.** In the thermal image **172** of the patient's right posterior calf, the thermal analyzer **70** analyzes to see how much red is in the thermal image. This may be suggestive of venous insufficiency, and the thermal analyzer **70** then provides a statement such as "this area has a 95% likelihood of having venous insufficiency."

The thermal analyzer **70** is advantageously configured to take advantage that there is a unique situation in venous disease where there are incompetent superficial veins, i.e., where the valves have failed. In legs with normal veins, for example, blood that fills the superficial veins is collected from the capillaries of the skin and subcutaneous fat.

Since the blood has come from the skin capillary network which acts as a heat exchanger, the temperature of blood filling the superficial veins is at the same temperature as the skin, and flows from distal to proximal and from superficial to deep.

As there is no thermal differential the veins are indistinguishable from the skin on thermal imaging. In contrast, where valves have become incompetent blood flows in the opposite direction, flowing from deep to superficial and from proximal to distal. In this instance blood refluxing from deep is at a core temperature of approximately 37°C, when it refluxes and fills the superficial veins the heat conducts to the skin. Consequently, an area overlying the varicose tributary is hotter than the typical skin temperature of around 30°C.

The sensitivity of thermal imaging may be as good as 0.1°C. This makes it easier to identify the hot areas. The hot areas are of course always losing heat energy, but as blood refluxes continuously during standing and activity this heat is replenished, and will remain hotter for a period after an individual is recumbent when reflux ceases.

Thus, a thermal image of the leg will show only veins with reflux and therefore failed valves, as hot areas that are linear in nature and serpiginous the highest temperature follows the course of the vein. This pattern may also be seen in arteriovenous fistula but these are rare and simply differentiated using ultrasound.

Thermal imaging does not identify incompetent trunk veins as they are deep to the deep fascia of the leg and have a uniform insulating subcutaneous layer. Thermal imaging thus uniquely demonstrates and differentiates legs with venous incompetence from normal limbs. Acquiring thermal images for venous disease does not require any preparation of the leg, is entirely non-invasive and very time efficient.

Referring back to operation of the clinical thermography application **52,** a side by side comparison between a thermal image **172** and a standard photograph **192** may be performed as provided in display screen **190** shown in FIG. 8. The display screen **190** includes the thermal image **172** of the patient's right leg and a standard photograph **192** of the patient's right leg as provided by the camera **58.** The thermal image **172** may be stored alongside the standard photograph **192** in the remote storage device **60.**

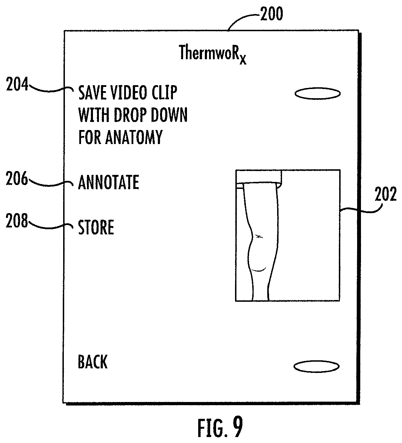

As an alternative to a thermal image, a thermal video clip **202** of the patient may be generated as provided in display screen **200** shown in FIG. 9. The same options available for a thermal image are available for a thermal video clip. The doctor has access to a save video clip field **204** for saving the thermal video clip **202.** The thermal video clip **202** is saved within the memory **51** prior to being stored in the remote storage device **60.**

The doctor has an option to annotate the thermal video clip **202** via the option to annotate field **206.** If the doctor wants to store the annotated thermal video clip **202** within the remote storage device **60,** then the store field **208** is selected. The thermal video clip **202** is stored within the cataloged patient thermal images **64.** At Block **132** the doctor quits or exits the clinical based thermography application **52.**

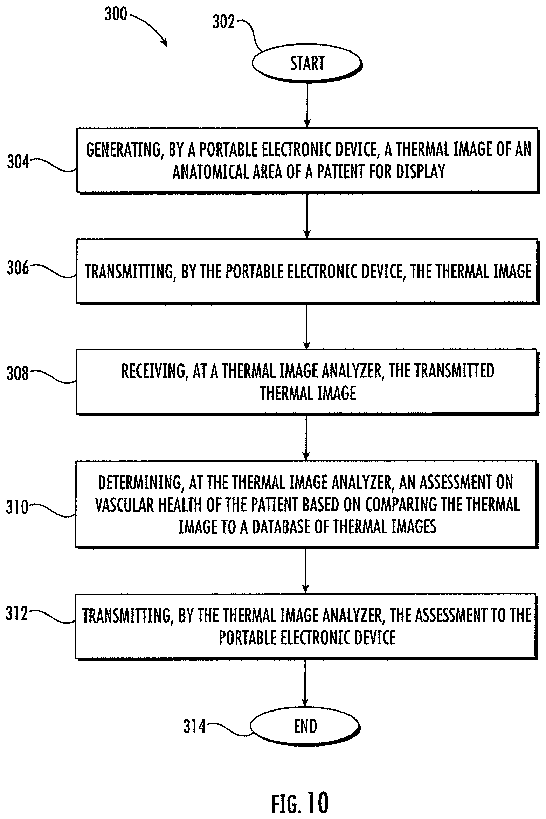

Referring now to FIG. 10, a flowchart **300** illustrating a method for operating the vascular thermography system **30** will be discussed. From the start (Block **302),** the method includes generating, by the portable electronic device **40,** a thermal image **172** of an anatomical area of a patient for display at Block **304.** The portable electronic device **40** transmits the thermal image at Block **306.** The method further includes receiving, at a thermal image analyzer **70,** the transmitted thermal image at Block **308.** The thermal image analyzer **70** determines an assessment on vascular health of the patient based on comparing the thermal image **174** to a database of thermal images **72** at Block **310.** The thermal image analyzer **70** then transmits the assessment to the portable electronic device **40** at Block **312.** The method ends at Block **314.**

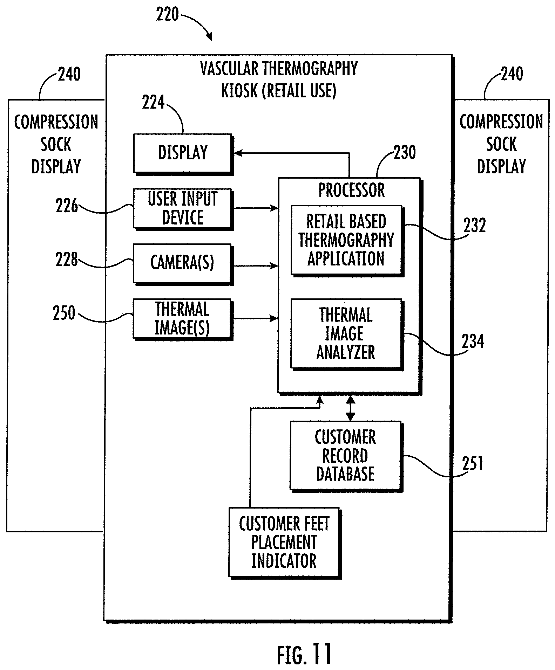

Referring now to FIG. 11 a retail based vascular thermography kiosk **220** will be discussed. The vascular thermography kiosk **220** may be placed in shopping malls or stores, for example, and is configured to assess the vascular health of a customer's legs so as to recommend appropriately sized compression socks as necessary.

The vascular thermography kiosk **220** includes a housing **222,** and one or more compression sock displays **240** attached to sides of the housing **222.** The housing **222** carries a display **224,** a user input device **226,** a processor **230,** at least one camera **228,** at least one thermal imager **250,** and a customer record database **251.** The vascular thermography kiosk **220** further includes a customer feet placement indicator **252.**

The customer feet placement indicator **252** allows a customer to stand on foot patterns that are in alignment with the thermal imager **250** and with the camera **228.** When the system has a fixed thermal imager **250,** the customer moves their feet to present the correct aspect to the thermal imager **250.** The placement of the feet is guided by foot prints on the floor placed at an optimal distance from the fixed camera **228.**

The thermal imager **250** generates one or more thermal images of the customer's legs, and the camera **228** also takes one or more picture of the customer's legs. The thermal imager **250** may be movable following a specific pattern to capture the relevant thermal images. Alternatively, there may be multiple thermal imagers **250** thereby avoiding the need for moving parts. The customer's legs are either bare or have tight clothing in contact with the skin, such as socks or stockings.

The thermal images may be displayed on the display **224** for customer viewing. Alternatively, the thermal images may be sent by internet to the customer and/or to a central location for storage and cataloging and review.

The thermal image will be processed to accentuate the hot vein pattern and according to the invention will subtract areas of normal heat, such as the anterior tibia area and popliteal fossa. The retail based thermography application **232** may further cooperate with the thermal imager **250** for setting a standard temperature scale and color pallet so that all images are comparable and consistent.

The thermal images and the photographs of the customer's legs may be stored photos in a customer record database **251.** Execution of the retail based thermography application **232** by the processor **230** provides display prompts on the display 224. The display prompts require the customer answering a series of clinical questions. For example, these questions may include the following: do you have leg swelling Y/N, do you have leg cramps Y/N, etc.

The customer's answers to the questions, along with the thermal image and photograph of the customer's legs are used by the thermal image analyzer 234 to give the customer suggestions regarding their venous health and whether or not certain types of compression socks might be most useful for the customer.

The thermal image analyzer 234 may be included within the processor 230 as shown, or may be a separate processor operating in coordination with the processor 230. In other embodiments, the thermal image analyzer 234 may be cloud based wherein the vascular thermography kiosk **220** then communicates with through the internet. The thermal image analyzer **234** may be configured to use machine learning to provide statistical probabilities of certain disease states based on the thermal patterns of the customer's thermal image. The thermal image analyzer **234** will apply the algorithms to determine for each leg a) are the leg vein normal or abnormal, and b) are compression garments indicated.

Operation of the retail based thermography application **232** allows the processor **230** to determine length and diameter of the customer's legs and thus determine which size of compression stocking is most appropriate. The vascular thermography kiosk **220** includes compression sock displays **240** for purchase based on the results of the analyses performed by the thermal image analyzer **234.**

As discussed above, the retail based vascular thermography kiosk **220** is a free standing autonomous system capable of deployment in public. The retail based vascular thermography kiosk **220** catalogs and stores customers' thermal images, and predicts the need for compression garments.

The retail based vascular thermography kiosk **220** is capable of producing a comprehensive map of a customer's incompetent venous circulation, and is capable of differentiating 'normal' areas of skin that are normally hotter than the general skin surface and disregarding them. The retail based vascular thermography kiosk **220** can in general determine normal from abnormal limbs (with regard to veins).

The thermal image analyzers **70, 234** may be based on artificial intelligence and machine learning. Veins of the leg, for example, are distributed in certain patterns and when they fail they do so in a variable recognizable number of patterns of varicose veins. Thermal imaging can detect these veins as broad areas of increased temperature above background.

The peak temperatures tend to follow the line of the vein which has a convoluted but linear form. Other areas of elevated temperature form a much more fixed pattern such as the area in the shin where the tibial bone is immediately beneath the skin, or behind the knee (the popliteal fossa, where subcutaneous fat is thinner).

In both these cases they do not create a linear pattern and so according to the invention an algorithm is developed to improve the specificity of the venous map by following the lines of maximum temperature and subtraction of area of diffuse high temperature. In this way a simplified but more accurate vein map can be created.

Knowledge of the absolute temperature range is also important. Body core temperature is 37°C, typical leg temperature of normal skin in the thigh at room temperature 20°C is between 28-32°C. Blood that issues from the deep is at or close to body temperature while blood that is in normal superficial veins is at the temperature of the skin.

The skin capillaries act as a very efficient heat exchanger and so the arterial blood which arrives to feed the skin is cooled imparting its heat to the skin before being collected into the veins by which time there is little or no temperature difference. This makes normal veins invisible to thermal imaging, as there is no temperature gradient between skin and vein.

In contrast to varicose veins, vein valves have failed and permit blood (when the patient is standing) to reflux to fill veins immediately beneath the skin (varicose veins) at close to core temperature with little insulating fat and so heat the skin over the vein to a temperature greater than the surrounding skin. This is easily within the discriminatory range of the thermal imaging camera. Controlling the temperature range of the thermal imager to between 28-38°C, or more preferably 30-36°C, improves sensitivity and specificity of vein detection.

Many modifications and other embodiments will come to the mind of one skilled in the art having the benefit of the teachings presented in the foregoing descriptions and the associated drawings. Therefore, it is understood that the disclosure is not to be limited to the specific embodiments disclosed, and that modifications and embodiments are intended to be included within the scope of the appended claims.

## Claims

1. A vascular thermography system comprising:
at least one portable electronic device (40) comprising:
a thermal imager (54) configured to generate a thermal image of an anatomical area of the leg of a patient with the thermal image being made from a differential of relative skin temperatures of an anatomical area of the leg of the patient,
a display (46),
a processor (50) configured to display the thermal image on said display, and
a transceiver (44) coupled to said processor and configured to transmit the thermal image;
a remote storage device (60) configured to store a cataloged thermal image (64) of the generated thermal image, wherein the thermal image is cataloged with corresponding label corresponding to anatomical area of the patient's leg, the thermal image and label being received from the portable electronic device; and
a thermal image analyzer (70) configured to perform the following:
receive the cataloged thermal image from said remote storage device,
determine an assessment on superficial vascular disease states of the patient's leg based on comparing the catalogued thermal image of the patient to a database of thermal images (72) with the database of thermal images comprising a plurality of thermal patterns corresponding to a range of superficial vascular disease states, by applying machine learning to a) determine thermal patterns within the thermal image, b) follow lines of maximum temperature and subtract areas of diffuse high temperatures based upon the thermal patterns, and c) provide statistical probabilities of superficial vascular disease states in the corresponding labeled anatomical area of the patient's leg based on the determined thermal pattern, and
transmit the assessment to said at least one portable electronic device.

2. The vascular thermography system according to Claim 1 wherein said remote storage device associated with said thermal image analyzer, said remote storage device configured to further store a patient list and the cataloged thermal images of patients on the patient list; and wherein said processor is further configured to execute a clinical thermography application to perform the following:
display a login screen; and
display vein scan settings based on a user of the at least one portable electronic device logging in, with the vein scan settings including at least one of the following:
a live view setting allowing the user to immediately examine a patient,
a new patient setting for the user to add a new patient to the patient list, and
an existing patient setting for the user to retrieve an existing patient from the patient list.

3. The vascular thermography system according to Claim 2 wherein said processor is further configured to perform the following based on execution of the clinical thermography application:
display a drop down list so the user can label the anatomical area being displayed in the thermal image; and
store the labeled thermal image in the cataloged thermal images at said remote storage device.

4. The vascular thermography system according to Claim 1 wherein said thermal image analyzer is cloud-based.

5. The vascular thermography system according to Claim 1 wherein communications between said at least one portable electronic device and said thermal image analyzer is encrypted.

6. The vascular thermography system according to Claim 1 wherein said at least one portable electronic device further comprises:
an external interface coupled to said processor; and
a housing configured to carry said display, said processor, said transceiver, and said external interface;
with said thermal imager being external said housing and removeably coupled to said external interface.

7. The vascular thermography system according to Claim 1 wherein said at least one portable electronic device further comprises a camera configured to take a photograph of the same anatomical area as being displayed in the thermal image; and wherein said processor is further configured to display in a side-by-side comparison the thermal image and the photograph of the same anatomical area.

## Patentansprüche

1. Gefäß-Thermografiesystem, umfassend:
mindestens ein tragbares elektronisches Gerät (40), umfassend:
eine Wärmebildkamera (54), die dazu eingerichtet ist, dass sie ein Wärmebild eines anatomischen Bereichs des Beins eines Patienten erzeugt, wobei das Wärmebild aus einer Differenz von relativen Hauttemperaturen eines anatomischen Bereichs des Beins des Patienten erstellt wird,
eine Anzeige (46),
einen Prozessor (50), der dazu eingerichtet ist, dass er das Wärmebild auf der Anzeige anzeigt und
einen Transceiver (44), der mit dem Prozessor verbunden und dazu eingerichtet ist, dass er das Wärmebild sendet;
einen externen Speicher (60), der dazu eingerichtet ist, dass er ein katalogisiertes Wärmebild (64) von dem erzeugten Wärmebild speichert, wobei das Wärmebild mit einer entsprechenden Bezeichnung katalogisiert ist, die dem anatomischen Bereich des Beins des Patienten entspricht, wobei das Wärmebild und die Bezeichnung von dem tragbaren elektronischen Gerät empfangen wird; und
einen Wärmebildanalysator (70), der dazu eingerichtet ist, Folgendes durchzuführen:
Empfangen des katalogisierten Wärmebilds von dem externen Speicher,
Bestimmen einer Beurteilung oberflächlicher Gefäßgesundheitszustände des Beins des Patienten anhand eines Vergleichs des katalogisierten Wärmebilds des Patienten mit einer Datenbank von Wärmebildern (72), wobei die Datenbank von Wärmebildern eine Mehrzahl von Wärmemustern umfasst, die einer Bandbreite von oberflächlichen Gefäßgesundheitszuständen entsprechen, indem maschinelles Lernen zum Einsatz kommt und damit a) Wärmemuster im Wärmebild ermittelt werden, b) auf Grundlage der Wärmemuster Linien mit der Höchsttemperatur gefolgt wird und Bereiche mit diffusen hohen Temperaturen abgezogen werden, und c) anhand des ermittelten Wärmemusters statistische Wahrscheinlichkeiten für oberflächliche Gefäßgesundheitszustände in dem entsprechenden mit einer Bezeichnung versehenen anatomischen Bereich des Beins des Patienten bereitgestellt werden, und
Senden der Beurteilung an das mindestens eine tragbare elektronische Gerät.

2. Gefäß-Thermografiesystem nach Anspruch 1, wobei der externe Speicher dem Wärmebildanalysator zugehörig ist, der externe Speicher dazu eingerichtet ist, dass er ferner eine Patientenliste und die katalogisierten Wärmebilder von Patienten in der Patientenliste speichert und wobei der Prozessor ferner dazu eingerichtet ist, dass er eine klinische Thermografieanwendung ausführt und damit Folgendes durchführt:
Anzeigen eines Anmeldebildschirms und
Anzeigen von Venenscaneinstellungen auf Grundlage eines Benutzers des mindestens einen tragbaren elektronischen Geräts, der sich anmeldet, wobei die Venenscaneinstellungen mindestens eins von Folgendem umfassen:
eine Live-Ansicht, die dem Benutzer eine unmittelbare Untersuchung eines Patienten ermöglicht,
eine Neupatient-Einstellung, mit der der Benutzer einen neuen Patienten zu der Patientenliste hinzufügen kann und
eine Bestandspatient-Einstellung, mit der der Benutzer einen Bestandspatienten von der Patientenliste abrufen kann.

3. Gefäß-Thermografiesystem nach Anspruch 2, wobei der Prozessor ferner dazu eingerichtet ist, auf Grundlage der Ausführung der klinischen Thermografieanwendung Folgendes durchzuführen:
Anzeigen einer Klappliste, damit der Benutzer den anatomischen Bereich, der in dem Wärmebild angezeigt wird, mit einer Bezeichnung versehen kann, und
Speichern des mit einer Bezeichnung versehenen Wärmebilds in den katalogisierten Wärmebildern in dem externen Speicher.

4. Gefäß-Thermografiesystem nach Anspruch 1, wobei der Wärmebildanalysator cloudbasiert ist.

5. Gefäß-Thermografiesystem nach Anspruch 1, wobei die Kommunikation zwischen dem mindestens einen tragbaren elektronischen Gerät und dem Wärmebildanalysator verschlüsselt ist.

6. Gefäß-Thermografiesystem nach Anspruch 1, wobei das mindestens eine tragbare elektronische Gerät ferner Folgendes umfasst:
eine externe Schnittstelle, die mit dem Prozessor verbunden ist, und
ein Gehäuse, das dazu eingerichtet ist, die Anzeige, den Prozessor, den Transceiver und die externe Schnittstelle zu tragen,
wobei sich die Wärmebildkamera außerhalb des Gehäuses befindet und abnehmbar mit der externen Schnittstelle verbunden ist.

7. Gefäß-Thermografiesystem nach Anspruch 1, wobei das mindestens eine tragbare elektronische Gerät ferner eine Kamera umfasst, die dazu eingerichtet ist, ein Foto von demselben anatomischen Bereich aufzunehmen, der in dem Wärmebild angezeigt wird, und wobei der Prozessor ferner dazu eingerichtet ist, zum Vergleich das Wärmebild und das Foto von ein und demselben anatomischen Bereich nebeneinander anzuzeigen.

## Revendications

1. Système de thermographie vasculaire comprenant :
au moins un dispositif portable électronique (40) comprenant :
un système d'imagerie thermique (54) configuré pour générer une image thermique d'une région anatomique de la jambe d'un patient, l'image thermique étant constituée d'un différentiel de températures relatives de la peau d'une région anatomique de la jambe du patient,
un affichage (46),
un processeur (50) configuré pour afficher l'image thermique sur ledit affichage, et
un émetteur-récepteur (44) couplé audit processeur et configuré pour transmettre l'image thermique ;
un dispositif de stockage à distance (60) configuré pour stocker une image thermique cataloguée (64) de l'image thermique générée, dans lequel l'image thermique est cataloguée avec une étiquette correspondante correspondant à la région anatomique de la jambe du patient, l'image thermique et l'étiquette étant reçues du dispositif électronique portable ; et
un analyseur d'images thermiques (70) configuré pour exécuter ce qui suit :
réception de l'image thermique cataloguée dudit dispositif de stockage à distance,
détermination d'une évaluation d'états de maladies vasculaires superficielles de la jambe du patient en se basant sur la comparaison de l'image thermique cataloguée du patient à une base de données d'images thermiques (72) avec la base de données d'images thermiques comprenant une pluralité de tracés thermiques correspondant à une plage d'états de maladies vasculaires superficielles, en appliquant un apprentissage machine pour a) déterminer des tracés thermiques à l'intérieur de l'image thermique, b) suivre des lignes de températures maximales et soustraire des régions de températures élevées diffuses en se basant sur les tracés thermiques, et c) fournir des probabilités statistiques d'états de maladies vasculaires superficielles dans la région anatomique étiquetée correspondante de la jambe du patient en se basant sur le tracé thermique déterminé, et
transmission de l'évaluation audit au moins un dispositif électronique portable.

2. Système de thermographie vasculaire selon la revendication 1, dans lequel ledit système de stockage à distance associé audit analyseur d'images thermiques, ledit dispositif de stockage à distance configuré pour stocker en outre une liste de patients et les images thermiques cataloguées de patients sur la liste de patients ; et dans lequel ledit processeur est en outre configuré pour exécuter une application de thermographie clinique pour exécuter ce qui suit :
afficher un écran de connexion ; et
afficher des réglages d'exploration de veines en se basant sur un utilisateur de l'au moins un dispositif électronique portable en train de se connecter, avec les réglages d'exploration de veines incluant au moins un de ce qui suit :
un réglage de vue en direct permettant à l'utilisateur d'examiner immédiatement un patient,
un réglage de nouveau patient pour que l'utilisateur ajoute un nouveau patient à la liste de patients, et
un réglage de patient existant pour que l'utilisateur retire un patient existant de la liste de patients.

3. Système de thermographie vasculaire selon la revendication 2, dans lequel ledit processeur est en outre configuré pour exécuter ce qui suit en se basant sur l'exécution de l'application de thermographie clinique :
afficher une liste déroulante pour que l'utilisateur puisse étiqueter la région anatomique en train d'être affichée dans l'image thermique ; et
stocker l'image thermique étiquetée dans les images thermiques cataloguées sur ledit dispositif de stockage à distance.

4. Système de thermographie vasculaire selon la revendication 1, dans lequel l'analyseur d'images est basé sur un cloud.

5. Système de thermographie vasculaire selon la revendication 1, dans lequel les communications entre ledit au moins un dispositif électronique portable et ledit analyseur d'images thermiques sont chiffrées.

6. Système de thermographie vasculaire selon la revendication 1, dans lequel ledit au moins un dispositif électronique portable comprend en outre :
une interface externe couplée audit processeur ; et
un logement configuré pour supporter ledit affichage, ledit processeur, ledit émetteur-récepteur et ladite interface externe ;
avec ledit système d'imagerie étant externe audit logement et couplé de manière amovible à ladite surface externe.

7. Système de thermographie vasculaire selon la revendication 1, dans lequel ledit au moins un dispositif électronique portable comprend en outre une caméra configurée pour prendre une photo de la même région anatomique telle qu'affichée dans l'image thermique ; et dans lequel ledit processeur est en outre configuré pour afficher dans une comparaison côte-à-côte l'image thermique et la photographie de la même région anatomique.
